# FASCICULE DE BREVET EUROPEEN

(11) **EP 0 788 491 B1**
(45) Date de publication et mention de la délivrance du brevet: **01.12.1999**
(21) Numéro de dépôt: 95935997.7
(22) Date de dépôt: 23.10.1995
(51) Int. Cl.: C07D 277/82, A61K 31/425

(54) **DERIVES DE 6-POLYFLUOROALCOXY ET 6-POLYFLUOROALKYLE-2-AMINOBENZOTHIAZOLE**
6-POLYFLUORALKOXY UND 6-POLYFLUORALKYL-2-AMINO BENZOTHIAZOLE-DERIVATE
6-POLYFLUOROALKOXY AND 6-POLYFLUOROALKYL-2-AMINOBENZOTHIAZOLE DERIVATIVES

(30) Priorité: 26.10.1994 FR 9412796
(43) Date de publication de la demande: 13.08.1997
(73) Titulaire: RHONE-POULENC RORER S.A., 92160 Antony (FR)
(72) Inventeur: CHAPELLE, Philip, F-91210 Draveil (FR); GAILLARD, Claude, F-94320 Thiais (FR); JIMONET, Patrick, F-78450 Villepreux (FR); LOUVEL, Erik, F-75010 Paris (FR); MARTINET, Michel, F-75012 Paris (FR); MIGNANI, Serge, F-92290 Châtenay-Malabry (FR); SANDERINK, Gérard, F-78470 Saint-Rémy-les-Chevreuses (FR)
(74) Mandataire: Savina, Jacques
(86) Numéro de dépôt international: FR9501392
(87) Numéro de publication internationale: WO9613492

(56) Documents cités:
- EP-A- 0 050 551
- EP-A- 0 374 040
- EP-A- 0 374 041

## Description

La présente invention concerne des composés de formule : 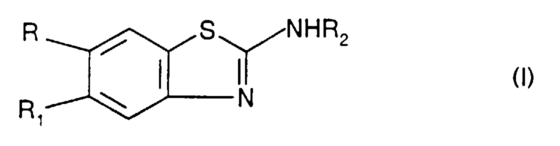 leurs sels, leur préparation et les médicaments les contenant.

Dans la formule (I), R représente un radical polyfluoroalcoxy ou polyfluoroalkyle,
et soit R₁ représente un atome d'hydrogène et R₂ représente un radical hydroxy,
soit R₁ représente un radical hydroxy et R₂ représente un atome d'hydrogène.

Les radicaux polyfluoroalcoxy sont de préférence les radicaux trifluorométhoxy, pentafluoroéthoxy, trifluoro-2,2,2 éthoxy et tétra-fluoro-1,1,2,2 éthoxy. Les radicaux polyfluoroalkyle sont de préférence des radicaux trifluorométhyle.

Dans les définitions qui précédent et celles qui seront citées ci-après, les radicaux et les portions alkyle et alcoxy contiennent 1 à 4 atomes de carbone en chaîne droite ou ramifiée.

Les composés de formule (I) pour lesquels R₁ représente un atome d'hydrogène et R₂ représente un radical hydroxy peuvent être préparés par action de chlorhydrate d'hydroxylamine sur un 2-chloro-6-polyfluoroalcoxy ou 6-polyfluoroalkyle-benzothiazole correspondant.

Cette réaction s'effectue au sein d'un solvant inerte tel qu'un alcool inférieur (méthanol par exemple), en présence d'une base telle qu'un hydroxyde de métal alcalin (soude, potasse par exemple), à la température d'ébullition du milieu réactionnel.

Les 2-chloro-6-polyfluoroalcoxy ou 6-polyfluoroalkyle-benzothiazoles peuvent être obtenus par application ou adaptation des méthodes décrites par S. MIGNANI et coll., Synth. Commun., 22 (19), 2769-2780 (1992) et dans les exemples.

Les composés de formule (I) pour lesquels R₁ représente un radical hydroxy et R₂ représente un atome d'hydrogène peuvent être préparés par déméthylation d'un 2-amino-5-méthoxy-6-polyfluoroalcoxy ou 6-polyfluoroalkyle-benzothiazole correspondant.

Cette déméthylation s'effectue généralement au moyen d'acide bromhydrique en excès, à la température d'ébullition du milieu réactionnel.

Les 2-amino-5-méthoxy-6-polyfluoroalcoxy ou 6-polyfluoroalkyle-benzothiazoles peuvent être obtenus par application ou adaptation des méthodes décrites dans les exemples.

Les mélanges réactionnels obtenus par les divers procédés décrits précédemment sont traités suivant des méthodes classiques physiques (évaporation, extraction, distillation, chromatographie, cristallisation....) ou chimiques (formation de sels....).

Les composés de formule (I) sous forme de base libre peuvent être éventuellement transformés en sels d'addition avec un acide minéral ou organique, par action d'un tel acide au sein d'un solvant organique tel qu'un alcool, une cétone, un éther ou un solvant chloré.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate.

EP-A-0 050 551 divulgue l'amino-2-trifluorométhoxy-6-benzothiazole comme anticonvulsant, anxiolitique et hypnotique.

EP-A-0 374 074 divulgue des dérivés de la benzothiazolamine-2 comme actifs vis-à-vis des convulsions induites par le glutamate.

Les composés de formule (I) et leurs sels présentent des propriétés pharmacologiques intéressantes. Ces composés interfèrent avec la transmission glutamatergique et sont donc utiles dans le traitement et la prévention des phénomènes liés au glutamate. Ces phénomènes sont notamment les manifestations épileptogènes et/ou convulsives, les troubles schizophréniques, les troubles du sommeil, l'anxiété, les phénomènes liés à l'ischémie cérébrale ainsi que les maladies neurodégénératives et les troubles neurologiques liés au vieillissement où le glutamate peut être impliqué tels que la maladie d'Alzheimer, la maladie de Parkinson, la maladie d'Huntington, la sclérose latérale amyotrophique, les traumas crâniens et médullaires et l'atrophie olivopontocérébelleuse.

L'activité de ces produits comme antiglutamate a été déterminée sur les convulsions induites par le glutamate selon une technique inspirée de celle de I. P. LAPIN, J. Neural. Transmission, 54, 229-238 (1982); l'injection du glutamate par voie intracérébroventriculaire étant effectuée selon une technique inspirée de celle de R. CHERMAT et P. SIMON, J. Pharmacol. (Paris), 6, 489-492 (1975). Leur DE₅₀ est inférieure à 10 mg/kg.

Les composés de formule (I) présentent une toxicité faible. Leur DL₅₀ est supérieure à 55 mg/kg par voie IP chez la souris.

Les composés de formule (I) préférés sont les suivants :
- 2-hydroxyamino-6-trifluorométhoxy-benzothiazole,
- 2-amino-5-hydroxy-6-trifluorométhoxy-benzothiazole,
- 2-hydroxyamino-6-trifluorométhyl-benzothiazole
et leurs sels.

Les exemples suivants illustrent l'invention.

### EXEMPLE 1

A 17,61 g de potasse en solution dans 350 ml de méthanol à une température voisine de 20°C, on ajoute 21,81 g de chlorhydrate d'hydroxylamine et on porte le milieu réactionnel à reflux pendant 10 minutes. 13,24 g de 2-chloro-6-trifluorométhoxy-benzothiazole sont alors ajoutés et la réaction poursuivie à reflux pendant 5 heures. Après refroidissement à une température voisine de 20°C, le précipité formé est filtré et le filtrat concentré à sec sous pression réduite (15 mm Hg, 2 kPa). Le produit brut ainsi obtenu est purifié par chromatographie flash sur colonne de silice en utilisant un mélange acétate d'éthyle-cyclohexane (20/80 en volumes) comme éluant. 7,35 g de 2-hydroxyamino-6-trifluorométhoxy-benzothiazole sont ainsi isolés sous forme de poudre jaune orangée fondant en se décomposant à 119°C [(Analyse % calculé C: 38,40; H: 2,01; F: 22,78; N: 11,20; S: 12,82; % trouvé C: 38,7; H: 1,9; F: 22,3; N: 11,1; S: 12,6); Spectre de R.M.N.¹H (250 MHz, (CD₃)₂SO d6, à une température de 373 K, δ en ppm) : 7,22 (mt, 2H : H 4 et H 5); 7,62 (s large, 1H : H 7); 9,55 et de 9,50 à 11,00 (respectivement s large et mf, 1H chacun : NHOH)].

Le 2-chloro-6-trifluorométhoxy-benzothiazole peut être préparé selon le procédé décrit par S. MIGNANI et coll., Synth. Commun., 22(19), 2769-2780 (1992).

### EXEMPLE 2

0,74 g de 2-amino-5-méthoxy-6-trifluorométhoxy-benzothiazole en solution dans 20 ml d'acide bromhydrique à 47% sont portés à reflux pendant 20 heures. Après refroidissement à une température voisine de 20°C, le milieu réactionnel est versé dans 20 ml d'eau glacée et la solution basifiée à l'aide de soude concentrée. La phase organique est alors extraite par de l'acétate d'éthyle, séchée sur sulfate de magnésium, filtrée et concentrée à sec sous pression réduite (15 mm Hg; 2 kPa). Le produit brut ainsi obtenu est purifié par chromatographie flash sur colonne de silice en utilisant un mélange dichlorométhane-méthanol (98/2 en volumes) comme éluant. 0,49 g de 2-amino-5-hydroxy-6-trifluorométhoxy-benzothiazole sont ainsi isolés sous forme de poudre blanche fondant à 216°C [(Analyse % calculé C : : 38,40; H : 2,01; F : 22,78; N : 11,20; S : 12,82; % trouvé C : 38,3; H : 1,9; F: 22,9; N : 11,2; S : 13,0); Spectre de R.M.N.¹H (250 MHz, (CD₃)₂SO d6, δ en ppm) : 6,96 (s large, 1H : H Aromatique en ortho du OH); 7,54 (s large, 2H : NH₂); 7,61 (s large, 1H : H Aromatique en méta du OH); 9,95 (mf, 1H : OH)].

Le 2-amino-5-méthoxy-6-trifluorométhoxy-benzothiazole peut être préparé selon la méthode suivante : à 0,8 g de 3-méthoxy-4-trifluorométhoxy-aniline en solution dans 10 ml d'acide acétique, on ajoute en une seule fois 1,5 g de thiocyanate de potassium. Après 20 minutes d'agitation à une température voisine de 20°C, une solution de 0,21 ml de brome dans 5 ml d'acide acétique est ajoutée goutte à goutte au milieu réactionnel en environ 30 minutes. La réaction est poursuivie 15 heures à la même température, puis le milieu réactionnel est dilué avec 15 ml d'eau distillée et neutralisé à l'aide de soude concentrée. La phase organique est extraite par de l'acétate d'éthyle, lavée à l'eau, séchée et concentrée à sec. Le produit brut ainsi obtenu est purifié par chromatographie flash sur colonne de silice en utilisant un mélange dichlorométhane-méthanol (90/10 en volumes) comme éluant. On obtient 0,74 g de produit attendu sous forme de poudre jaune fondant à 153°C [Spectre de R.M.N.¹H (300 MHz, (CD₃)₂SO d6, δ en ppm) : 3,87 (s, 3H : OCH₃); 7,16 (s, 1H : H Aromatique en ortho du OCH₃); 7,62 (s large, 2H : NH₂); 7,73 (s large, 1H : H Aromatique en méta du CH₃)].

La 3-méthoxy-4-trifluorométhoxy-aniline peut être obtenue selon le protocole suivant : 0,5 g de 3-méthoxy-4-trifluorométhoxy-nitrobenzène, en solution dans 10 ml de méthanol, sont hydrogénés à pression atmosphèrique et à une température voisine de 20°C, en présence de 10 mg d'oxyde de platine. Après absorption du volume théorique en hydrogène, le milieu réactionnel est filtré sur célite et le filtrat concentré à sec sous pression réduite. Le produit brut est purifié par filtration sur silice en utilisant un mélange acétate d'éthyle-cyclohexane (50/50 en volumes) comme éluant. On obtient ainsi 0,4 g de produit attendu sous forme d'huile jaune [Spectre de R.M.N.¹H (200 MHz, (CD₃)₂SO d6, δ en ppm) : 3,76 (s, 3H : OCH₃); 5,33 (s large, 2H : NH₂); 6,16 (dd, J = 9 et 2,5 Hz, 1H : H Aromatique en para du OCH₃); 6,39 (d, J = 2,5 Hz, 1H : H Aromatique en ortho du OCH₃); 6,96 (d large, J = 9 Hz, 1H : H Aromatique en méta du OCH₃)].

Le 3-méthoxy-4-trifluorométhoxy-nitrobenzène peut être préparé selon la procédure suivante : à 1,3 g de 2-trifluorométhoxy-5-nitrophénol dans 6 ml d'éthanol portés à 50°C, on ajoute 0,66 ml de diméthylsulfate, puis une solution aqueuse de soude préparée à partir de 0,28 g de soude en pastilles et 3 ml d'eau distillée. Le milieu réactionnel est chauffé à reflux pendant 4 heures. Après refroidissement à une température voisine de 20°C, 10 ml d'eau distillée sont ajoutés au milieu réactionnel et la phase organique extraite par du dichlorométhane. Le produit brut obtenu par traitement habituel est purifié par chromatographie flash sur colonne de silice en utilisant un mélange dichlorométhane-cyclohexane (20/80 en volumes) comme éluant. On obtient ainsi 0,5 g de produit attendu sous forme d'huile jaune [Spectre de R.M.N.¹H (200 MHz, (CD₃)₂SO d6, δ en ppm): 4,01 (s, 3H: OCH₃); 7,70 (d large, J = 9 Hz, 1H : H Aromatique en méta du OCH₃); 7,96 (dd, J = 9 et 2,5 Hz, 1H : H Aromatique en para du OCH₃); 8,04 (d, J = 2,5 Hz, 1H : H Aromatique en ortho du OCH₃)].

Le 2-trifluorométhoxy-5-nitrophénol peut être préparé selon la méthode suivante : à une suspension de 2,35 g de 2-trifluorométhoxy-5-nitroaniline dans 17 ml d'eau distillée refroidie à 5°C et en présence de 5 ml d'acide chlorhydrique concentré, on ajoute goutte à goutte une solution aqueuse (2 ml) de nitrite de sodium (0,82 g). Après 30 minutes d'agitation à la même température, on ajoute, goutte à goutte au milieu réactionnel devenu limpide, une solution aqueuse (5 ml) de tétrafluoroborate de sodium (1,66 g). Après 30 minutes de réaction toujours à la même température, le précipité formé est filtré, lavé à l'eau et séché. On obtient ainsi 1,85 g de tétrafluoroborate de 2-trifluorométhoxy-5-nitrophényldiazonium sous forme de poudre blanche utilisée sans purification supplémentaire dans les synthèses ultérieures. Ce sel de diazonium est ensuite partagé en 4 lots d'environ 0,5 g. Chaque lot est ajouté à une solution de 150 g de nitrate de cuivre (trihydrate) dans 100 ml d'eau distillée. 0,15 g d'oxyde de cuivre sont alors ajoutés et la réaction poursuivie 30 minutes à une température voisine de 20°C. Après filtration de l'insoluble sur un lit de célite et lavage avec du dichlorométhane, la phase organique est extraite du filtrat par du dichlorométhane et les 4 phases organiques regroupées et concentrées à sec sous pression réduite. Le produit brut (1 g d'huile brune) est purifié par chromatographie flash sur colonne de silice en utilisant un mélange acétate d'éthyle-cyclohexane (10/90 en volumes) comme éluant. On obtient ainsi 0,4 g de phénol attendu sous forme de poudre jaune pâle [Spectre de R.M.N.¹H (200 MHz, (CD₃)₂SO d6, δ en ppm) : 7,61 (d large, J = 9 Hz, 1H : H Aromatique en méta du OH); 7,78 (dd, J = 9 et 2,5 Hz, 1H : H Aromatique en para du OH); 7,86 (d, J = 2,5 Hz, 1H : H Aromatique en ortho du OH); 11,38 (mf étalé, 1H : OH)].

La 2-trifluorométhoxy-5-nitroaniline peut être préparée selon le protocole suivant : à 280 ml d'acide sulfurique concentré refroidis à 0°C, on ajoute progressivement 10 g de 2-trifluorométhoxyaniline, puis par petites portions 5,7 g de nitrate de potassium. La réaction est poursuivie 2 heures à la même température. Le milieu réactionnel est alors coulé sur 1 litre de glace puis dilué avec 3 litres d'eau distillée. L'extraction de la phase organique par trois fois 500 ml d'éther éthylique conduit après séchage sur sulfate de magnésium, filtration et concentration à sec sous pression réduite à 10,9 g de produit attendu sous forme de solide jaune fondant à 88°C [Spectre de R.M.N.¹ H (200 MHz, CDCl₃, δ en ppm) : 4,00 (mf, 2H : NH₂) ; 7,18 (d large, J = 9 Hz, 1H : H Aromatique en méta du NH₂); 7,52 (dd, J = 9 et 2,5 Hz, 1H : H Aromatique en para du NH₂); 7,60 (d, J = 2,5 Hz, 1H : H Aromatique en ortho du NH₂)].

### EXEMPLE 3

A une solution de 0,7 g de potasse dans 15 ml de méthanol, on ajoute 0,88 g de chlohydrate d'hydroxylamine et on porte le milieu réactionnel au reflux pendant 10 minutes. On ajoute alors 0,5 g de 2-chloro-6-trifluorométhyl-benzothiazole et on poursuit le reflux pendant 8 heures. Après refroidissement à une température voisine de 20°C, l'insoluble est filtré, le filtrat concentré à sec et le résidu repris dans le dichlorométhane. La phase organique est lavée à l'eau, puis séchée et concentrée à sec sous pression réduite (15 mm Hg, 2 kPa). Le produit brut ainsi obtenu est purifié par chromatographie flash sur colonne de silice en utilisant un mélange acétate d'éthyle-cyclohexane (40/60 en volumes) comme éluant. On obtient ainsi 0,35 g de 2-hydroxyamino-6-trifluorométhyl-benzothiazole sous forme de poudre orangée dont le point de fusion est supérieur à 260°C (Analyse % calculé C : 41,03; H : 2,15; N : 11,96; S : 13,69; trouvé C : 41,4; H : 1,6; N :12,3; S : 13,9) [Spectre de R.M.N.¹H (200 MHz, (CD₃)₂SO d6, δ en ppm) : 7,2 (s large, 1H : CH aromatique), 7,53 (d, J = 7Hz : CH aromatiques, 8,0 (s large, 1H : CH aromatiques), 10,07 (s, 1H : NH, 11,05 (s, 1H : OH)].

Le 2-chloro-6-trifluorométhyl-benzothiazole peut être préparé selon le protocole suivant : à une solution de 5 g de 2-amino-6-trifluorométhyl-benzothiazole dans 17 ml d'acide chlorhydrique 6N refroidi à 0°C, on ajoute goutte à goutte, une solution de 3 g de nitrite de sodium dans 5 ml d'eau distillée et on poursuit l'agitation à la même température pendant 2 heures. Le milieu réactionnel est alors traité par 13,8 g de chlorure cuivreux et la réaction prolongée 4 heures à une température voisine de 20°C. Le milieu réactionnel est alors versé sur 150 ml d'eau glacée et l'insoluble filtré puis repris dans le dichlorométhane et le nouvel insoluble écarté. Le filtrat est lavé à l'eau puis cette phase organique est séchée et concentrée à sec sous pression réduite (15 mmHg; 2 kPa). Le produit brut est purifié par chromatographie flash sur colonne de silice en utilisant un mélange cyclohexane-dichlorométhane (95/5 en volumes) comme éluant. On obtient ainsi 1,47 g de produit attendu sous forme de meringue jaune [Spectre de R.M.N.¹H (300 MHz, (CD₃)₂SO d6, δ en ppm) : 7,91 (d, J = 7Hz, 1H : CH aromatique), 8,20 (d, J = 7Hz, 1H : CH aromatiques), 8,7 (s, 1H : CH aromatique)]

Le 2-amino-6-trifluorométhyl-benzothiazole peut être obtenu selon la méthode décrite dans le brevet US 2822359.

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, intraveineuse, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention interfèrent avec la transmission glutamatergique et sont donc particulièrement utiles dans le traitement et la prévention des désordres liés au glutamate. Ces composés sont notamment utiles pour le traitement ou la prévention des manifestations épileptogènes et/ou convulsives, des troubles schizophréniques, des troubles du sommeil, de l'anxiété, des phénomènes liés à l'ischémie cérébrale ainsi que les troubles neurodégénérateurs et les troubles neurologiques liés au vieillissement où le glutamate peut être impliqué telles que la maladie d'Alzheimer, la maladie de Parkinson, la maladie d'Huntington, la sclérose latérale amyotrophique, les traumas crâniens et médullaires et l'atrophie olivopontocérébelleuse.

Les doses dépendent de l'effet recherché, de la durée du traitement et de la voie d'administration utilisée; elles sont généralement comprises entre 30 et 300 mg par jour par voie orale pour un adulte avec des doses unitaires allant de 10 mg à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle de comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 cm3
- Benzoate de sodium 80 mg
- Ethanol à 95 % 0,4 cm3
- Hydroxyde de sodium 24 mg
- Propylène glycol 1,6 cm3
- Eau q.s.p. cm3

## Revendications

1. Composés de formule : 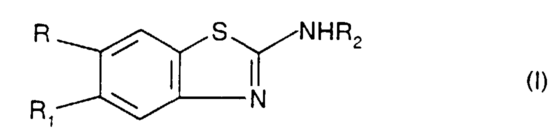 dans laquelle R représente un radical polyfluoroalcoxy ou polyfluoroalkyle,
et soit R₁ représente un atome d'hydrogène et R₂ représente un radical hydroxy,
soit R₁ représente un radical hydroxy et R₂ représente un atome d'hydrogène
étant entendu que les portions alcoxy et alkyle contiennent 1 à 4 atomes de carbone en chaîne droite ou ramifiée et leurs sels.

2. Composés selon la revendication 1 pour lesquels R représente un radical trifluorométhoxy, pentafluoroéthoxy, trifluoro-2,2,2 éthoxy, tétra-fluoro-1,1,2,2 éthoxy ou trifluorométhyle.

3. Composés de formule (I) suivants :
- 2-hydroxyamino-6-trifluorométhoxy-benzothiazole,
- 2-amino-5-hydroxy-6-trifluorométhoxy-benzothiazole
- 2-hydroxyamino-6-trifluorométhyle-benzothiazole
et leurs sels.

4. Procédé de préparation des composés de formule (I) selon la revendication 1 pour lesquels R₁ représente un atome d'hydrogène et R₂ représente un radical hydroxy caractérisé en ce que l'on fait réagir le chlorhydrate d'hydroxylamine sur un 2-chloro-6-polyfluoroalcoxy ou 6-polyfluoroalkyle-benzothiazole correspondant, isole le produit et le transforme éventuellement en sel.

5. Procédé de préparation des composés de formule (I) selon la revendication 1 pour lesquels R₁ représente un radical hydroxy et R₂ représente un atome d'hydrogène caractérisé en ce que l'on déméthyle un 2-amino-5-méthoxy-6-polyfluoroalcoxy ou 6-polyfluoroalkyle-benzothiazole correspondant, isole le produit et le transforme éventuellement en sel.

6. Médicaments contenant en tant que principe actif au moins un composé de formule (I) selon la revendication 1 ou un sel d'un tel composé.

7. Médicaments contenant en tant que principe actif au moins un composé selon la revendication 2 ou un sel d'un tel composé.

8. Médicaments contenant en tant que principe actif au moins un composé selon la revendication 3 ou un sel d'un tel composé.

9. Médicaments selon l'une des revendications 6 à 8 utiles comme antiglutamates.

10. Médicaments selon l'une des revendications 6 à 8 pour le traitement ou la prévention des manifestations épileptogènes et/ou convulsives, des troubles schizophréniques, des troubles du sommeil, de l'anxiété, des phénomènes liés à l'ischémie cérébrale, la maladie d'Alzheimer, la maladie de Parkinson, la maladie d'Huntington, la sclérose latérale amyotrophique, les traumas crâniens et médullaires et l'atrophie olivopontocérébelleuse.

## Patentansprüche

1. Verbindungen der Formel: 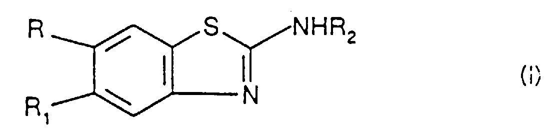 in der R einen Polyfluoralkoxy- oder Polyfluoralkylrest darstellt,
und entweder R₁ ein Wasserstoffatom darstellt und R₂ einen Hydroxyrest darstellt,
oder R₁ einen Hydroxyrest darstellt und R₂ ein Wasserstoffatom darstellt,
wobei es sich versteht, daß die Alkoxy- und Alkylteile 1 bis 4 Kohlenstoffatome in gerader oder verzweigter Kette enthalten,
und ihre Salze.

2. Verbindungen gemäß Anspruch 1, für die R einen Trifluormethoxy-, Pentafluorethoxy-, 2,2,2-Trifluorethoxy-, 1,1,2,2-Tetrafluorethoxy- oder Trifluormethylrest darstellt.

3. Folgende Verbindungen der Formel (I):
- 2-Hydroxyamino-6-trifluormethoxy-benzothiazol
- 2-Amino-5-hydroxy-6-trifluormethoxy-benzothiazol
- 2-Hydroxyamino-6-trifluormethyl-benzothiazol
und ihre Salze.

4. Verfahren zur Herstellung der Verbindungen der Formel (I) gemäß Anspruch 1, für die R₁ ein Wasserstoffatom darstellt und R₂ einen Hydroxyrest darstellt, dadurch gekennzeichnet, daß man Hydroxylamin-Chlorhydrat mit einem entsprechenden 2-Chlor-6-polyfluoralkoxy- oder -6-polyfluoralkyl-benzothiazol umsetzt, das Produkt isoliert und es gegebenenfalls in ein Salz überführt.

5. Verfahren zur Herstellung der Verbindungen der Formel (I) gemäß Anspruch 1, für die R₁ einen Hydroxyrest darstellt und R₂ ein Wasserstoffatom darstellt, dadurch gekennzeichnet, daß man ein entsprechendes 2-Amino-5-methoxy-6-polyfluoralkoxy- oder -6-polyfluoralkyl-benzothiazol demethyliert, das Produkt isoliert und es gegebenenfalls in ein Salz überführt.

6. Medikamente, die als Wirkstoff wenigstens eine Verbindung der Formel (I) gemäß Anspruch 1 oder ein Salz einer solchen Verbindung enthalten.

7. Medikamente, die als Wirkstoff wenigstens eine Verbindung gemäß Anspruch 2 oder ein Salz einer solchen Verbindung enthalten.

8. Medikamente, die als Wirkstoff wenigstens eine Verbindung gemäß Anspruch 3 oder ein Salz einer solchen Verbindung enthalten.

9. Medikamente gemäß einem der Ansprüche 6 bis 8, die als Antiglutamate verwendbar sind.

10. Medikamente gemäß einem der Ansprüche 6 bis 8 für die Behandlung oder zur Vorbeugung von epileptischen und/oder konvulsiven Erscheinungen, von schizophrenen Störungen, von Schlafstörungen, von Angstzuständen, von Phänomenen, die mit der zerebralen Ischämie, der Alzheimer-Krankheit, der Parkinson-Krankheit, der Huntington-Krankheit, der amyotrophischen Lateralsklerose, den Schädel- und Rückenmarkstraumen und der olivo-pontozerebellaren Atrophie verbunden sind.

## Claims

1. Compounds of formula: 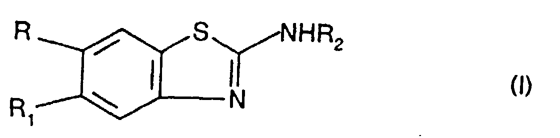 in which R represents a polyfluoroalkoxy or polyfluoroalkyl radical,
and either R₁ represents a hydrogen atom and R₂ represents a hydroxyl radical,
or R₁ represents a hydroxyl radical and R₂ represents a hydrogen atom
it being understood that the alkoxy and alkyl portions contain 1 to 4 straight- or branched-chain carbon atoms,
and their salts.

2. Compounds according to claim 1, in which R represents a trifluoromethoxy, pentafluoroethoxy, 2,2,2-trifluoroethoxy, 1,1,2,2-tetrafluoroethoxy or trifluoromethyl radical.

3. Following compounds of formula (I):
- 2-hydroxyamino-6-(trifluoromethoxy)benzothiazole,
- 2-amino-5-hydroxy-6-(trifluoromethoxy)benzothiazole,
- 2-hydroxyamino-6-(trifluoromethyl)benzothiazole
and their salts.

4. Process for the preparation of the compounds of formula (I) according to claim 1 in which R₁ represents a hydrogen atom and R₂ represents a hydroxyl radical, characterized in that hydroxylamine hydrochloride is reacted with a corresponding 2-chloro-6-(polyfluoroalkoxy)- or -6-(polyfluoroalkyl)- benzothiazole, the product is isolated and is optionally converted to a salt.

5. Process for the preparation of the compounds of formula (I) according to claim 1 in which R₁ represents a hydroxyl radical and R₂ represents a hydrogen atom, characterized in that a corresponding 2-amino-5-methoxy-6-(polyfluoroalkoxy)- or -6- (polyfluoroalkyl)benzothiazole is demethylated, the product is isolated and is optionally converted to a salt.

6. Medicaments containing, as active principle, at least one compound of formula (I) according to claim 1 or a salt of such a compound.

7. Medicaments containing, as active principle, at least one compound according to claim 2 or a salt of such a compound.

8. Medicaments containing, as active principle, at least one compound according to claim 3 or a salt of such a compound.

9. Medicaments according to one of claims 6 to 8 which are useful as antiglutamates.

10. Medicaments according to one of claims 6 to 8 for the treatment or the prevention of epileptogenic and/or convulsive manifestations, of schizophrenic disorders, of sleep disorders, of anxiety, of phenomena related to cerebral ischaemia, Alzheimer's disease, Parkinson's disease, Huntington's chorea, amyotrophic lateral sclerosis, cranial and medullary traumas and olivopontocerebellar atrophy.
